# EUROPEAN PATENT APPLICATION

(11) **EP 1 469 083 A1**
(43) Date of publication of application: **20.10.2004**
(21) Application number: 03290958.2
(22) Date of filing: 17.04.2003
(51) Int. Cl.: C12Q 1/68

(54) **Method of calibration of reverse transcription using a synthetic messenger RNA (smRNA) as an internal control**

(71) Applicant: CENTRE NATIONAL DE LA RECHERCHE SCIENTIFIQUE (CNRS), 75016 Paris (FR); UNIVERSITE CLAUDE BERNARD - LYON 1, F-69622 Villeurbanne Cédex (FR)
(72) Inventor: Morales, Anne Catherine, 69500 Bron (FR); Bezin, Laurent Georges Bernard, 69005 Lyon (FR)
(74) Representative: Le Forestier, Eric

(57) **Abstract**

The invention pertains to the field of reverse transcription reactions and relates to a new method of calibration with a synthetic messenger RNA (smRNA). It is aimed at a synthetic messenger RNA (smRNA) designed for normalization of reverse transcription reaction of mRNAs of a biological sample, wherein said smRNA do not interfere with the reverse transcription of endogenous mRNA of said sample.

SEQUENCE LISTING

<110> CENTRE NATIONAL DE LA RECHERCHE SCIENTIFIQUE - CNRS

<120> Method of calibration of reverse transcription using a synthetic
      messenger RNA (smRNA)

<130> D21194

<160> 18

<170> PatentIn version 3.2

<210> 1
<211> 161
<212> RNA
<213> Artificial

<220>
<223> Synthetic poly A mRNA #1

<400> 1
gggcgaauug ggcccgacgu cgggacaaga agguggaaga cgucaugcuc ccggccgcca      60
uggcggccgc gggaauucga uuucuucgac ucacugcaga cuacugaugg aaugacguag     120
uacgaauacu cgacuggucu caacaugaaa aaaaaaaaaa a                         161

<210> 2
<211> 161
<212> RNA
<213> Artificial

<220>
<223> Synthetic poly A mRNA #2

<400> 2
uaauacgacu cacuauaggg cgggacaaga agguggaaga cgucaugcuc ccggccgcca      60
uggcggccgc gggaauucga uuucuucgac ucacugcaga cuacugaugg aaugacguag     120
uacgaauacu cgacuggucu caacaugaaa aaaaaaaaaa a                         161

<210> 3
<211> 161
<212> DNA
<213> Artificial

<220>
<223> Synthetic cDNA #1

<400> 3
gggcgaattg ggcccgacgt cgggacaaga aggtggaaga cgtcatgctc ccggccgcca      60
tggcggccgc gggaattcga tttcttcgac tcactgcaga ctactgatgg aatgacgtag     120
tacgaatact cgactggtct caacatgaaa aaaaaaaaaa a                         161

<210> 4
<211> 161
<212> DNA
<213> Artificial

<220>

<223> Synthetic cDNA #2

<400> 4
taatacgact cactataggg cgggacaaga aggtggaaga cgtcatgctc ccggccgcca 60
tggcggccgc gggaattcga tttcttcgac tcactgcaga ctactgatgg aatgacgtag 120
tacgaatact cgactggtct caacatgaaa aaaaaaaaaa a 161


<210> 5
<211> 19
<212> DNA
<213> Artificial

<220>
<223> Primer III forward

<400> 5
cgggacaaga aggtggaag 19


<210> 6
<211> 22
<212> DNA
<213> Artificial

<220>
<223> Primer III reverse

<400> 6
agtctgcagt gagtcgaaga aa 22


<210> 7
<211> 182
<212> DNA
<213> Artificial

<220>
<223> Sequence of the DNA probe "DNAΣ"

<400> 7
taatacgact cactataggg cgggacaaga aggtggaaga cgtcatgctc ccggccgcca 60
tggcggccgc gggaattcga tttcttcgac tcactgcaga ctactgatgg aatgacgtag 120
tacgaatact cgactggtct caacatgaaa aaaaaaaaaa acgcattcaa cctgtctgac 180
ta 182


<210> 8
<211> 20
<212> DNA
<213> Artificial

<220>
<223> Sequence of the T7 promoter

<400> 8
taatacgact cactataggg 20


<210> 9
<211> 27

<212> DNA
<213> Artificial

<220>
<223> 27 pb insert

<400> 9
cgggacaaga aggtggaaga cgtcatg 27

<210> 10
<211> 34
<212> DNA
<213> Artificial

<220>
<223> 34 bp from pGEM®-T Easy sequence

<400> 10
ctcccggccg ccatggcggc cgcgggaatt cgat 34

<210> 11
<211> 101
<212> DNA
<213> Artificial

<220>
<223> 101 bp insert

<400> 11
ttcttcgact cactgcagac tactgatgga atgacgtagt acgaatactc gactggtctc 60
aacatgaaaa aaaaaaaaaa cgcattcaac ctgtctgact a 101

<210> 12
<211> 39
<212> DNA
<213> Artificial

<220>
<223> Forward primer A containing the T7 promoter

<400> 12
taatacgact cactataggg cgggacaaga aggtggaag 39

<210> 13
<211> 21
<212> DNA
<213> Artificial

<220>
<223> Reverse primer A

<400> 13
tagtcagaca ggttgaatgc g 21

<210> 14
<211> 82

<212> DNA
<213> Artificial

<220>
<223> Amplified fragment from both synthetic cDNA #1 and cDNA #2 with
      primer pair III

<400> 14
cgggacaaga aggtggaaga cgtcatgctc ccggccgcca tggcggccgc gggaattcga      60
tttcttcgac tcactgcaga ct                                                82


<210> 15
<211> 20
<212> DNA
<213> Artificial

<220>
<223> Pair I: primer forward

<400> 15
aattgggccc gacgtcgcat                                                   20


<210> 16
<211> 20
<212> DNA
<213> Artificial

<220>
<223> Pair I: primer reverse

<400> 16
catgttgaga ccagtcgagt                                                   20


<210> 17
<211> 19
<212> DNA
<213> Artificial

<220>
<223> Pair II: primer forward

<400> 17
cgggacaaga aggtggaag                                                    19


<210> 18
<211> 20
<212> DNA
<213> Artificial

<220>
<223> Pair II: primer reverse

<400> 18
tcatgttgag accagtcgag                                                   20

## Description

The invention pertains to the field of reverse transcription reactions and relates to a new method of calibration with a synthetic messenger RNA (smRNA).

Quantitative RT-PCR assays involve simultaneously amplifying control molecules and samples containing a target mRNA. Known amounts of a control molecule are thermally cycled with tubes, wells or slides, containing the unknown amount of target. In addition to the pair of PCR primers for the target, a pair of PCR primers is required for the control molecule. Following amplification, the amounts of amplified products are compared. However, inherent variation in amplification efficiency among primers results in poor quality of results.

More recently, this method has been improved. Kits comprising fluorescent dyes and quenchers are provided by Applied Biosystems and are available under the trademark TaqMan™. Improvements allowed the development of real-time quantitative PCR.

These methods have been reviewed for example in Heid et al, Real-time quantitative PCR. Genome Research. 1996; 6: 986-994, and in Gibson et al, Genome Research, A novel method for real time quantitative RT-PCR Genome research. 1996; 6 : 995-1001. Despite the use of different sets of tubes containing triplicate two-fold dilutions of a control molecule and a fixed amount of unknown target, variation in the quantification between samples still occurs.

We found in connection with the invention that such drawback is due to the nature of the control molecule used. In Heid et al, the internal standard used for normalization is an endogenous gene such as β-actin. Endogenous genes are not appropriate since they may vary from one sample to the other and can interfere with amplification of related gene sequences. In Gibson et al, it is proposed to use a random sequence selected from the target sequence or a modified sequence deriving from the target sequence. In this case, variation between sample may also occurs and it would require the design of one control molecule for each target sequence, which makes it impossible when analyzing the expression of large number of genes. In Wang et al, Quantification of mRNA by the polymerase chain reaction, Proc. Acad. Sci. USA, 1989, 86 : 9717-9721, it is suggested to use a standard whose sequence comprise complementary sequence of the target gene so that one pair of primer is sufficient to amplify both products. Again, such approach is irrelevant with the analysis of a large number of genes. A similar method is described in WO 00/20629.

Because quantitative PCR amplifications (real-time PCR) is now widely used for the quantification of the relative alteration in mRNA expression, controlling the RT reaction efficiency using real-time PCR has become an important factor before the further use of the RT products (microarrays, DD-RTPCR ...).

We provide a solution to address the above mentioned problems based on the use of a synthetic messenger RNA (smRNA) designed for normalization of reverse transcription reaction of mRNAs of a biological sample, which smRNA do not interfere with the reverse transcription of endogenous mRNA of said sample. Such smRNA is particularly advantageous for the analysis of different genes from different samples for example using real-time quantitative RT-PCR.

### Description

Therefore, in a first aspect, the invention relates to a method for quantifying products of reverse transcription reaction of mRNA extracted from a biological sample, wherein said method comprises the steps consisting of adding a synthetic messenger RNA (smRNA) which do not interfere with the reverse transcription of endogenous mRNA of said sample to a reaction mixture comprising mRNA extracted from said sample, which smRNA display less than 95%, 97% or 99% or preferably less than 99,50 % identity with any biological sequence, to perform a reverse transcription reaction and to calibrate the quantity of each target mRNA obtained after amplification with the quantity of the cDNA amplified corresponding to said smRNA.

In this method, said smRNA can be added in different reaction mixtures comprising one sample in different dilutions.

Said smRNA may further comprise a polyA segment. This allows to perform the reverse transcriptase reaction using a polydT primer for both the target mRNAs and the smRNA.

The smRNA may be about 80 to 150 nucleotide long or longer, preferably about 100 nucleotide long and comprise particular sequences so as to be specifically amplified with a pair of primers designed to avoid primer dimerization.

The method depicted above may consist of quantitative RT-PCR, DD-RT-PCR, wherein quantification of target mRNAs is performed by means of normalization with said smRNA. The invention can be practiced on DNA microarrays. In this case, the cDNA corresponding to the smRNA is amplified and spotted onto said microarrays. This is an important advantage of the said mRNA, since a same mRNA sequence can be used first to help calibrating the RT reaction in microarrays, and second, to calibrate the RT reaction in real-time RT-PCR (which is a commonly used technique employed following microarrays to validate the results obtained). In other words, both techniques (microarrays and real-time RT-PCR) can use a same standard to control the reaction's efficiency.

Alternatively, the method of the invention may consist of Northern blotting wherein said smRNA is pooled with samples.

In a particular embodiment, the smRNA consists of a SEQ ID No 1, SEQ ID No 2, or sequences deriving thereof. It is reversed transcribed into cDNA of SEQ ID No 3 or SEQ ID No 4, preferably using a pair of primer consisting of SEQ ID No 5 and SEQ ID No 6. Such primers are optimized so as to avoid dimerization and are specific for the smRNA sequence.

In a second embodiment, the invention relates to a synthetic messenger RNA (smRNA) designed for normalization of reverse transcription reaction of mRNAs of a biological sample, wherein said smRNA do not interfere with the reverse transcription of endogenous mRNA of said sample, which smRNA display less than 95% or 99% identity with any biological sequence, comprise a poly A segment and is about 80 nucleotide long or longer, preferably about 100 nucleotide long.

This smRNA may further comprise at least two segments which are complementary to at least two primers that are designed to avoid primer dimerization.

One preferred smRNA has the sequence shown in SEQ ID No 1 or SEQ ID No 2.
Another feature of these particular smRNA is two segments which are complementary to two primers of SEQ ID No 5 and SEQ ID No 6.
In this regards, the invention is also directed to a primer or probe selected from of SEQ ID No 5 and SEQ ID No 6.

The invention also encompasses a cDNA obtained from a reverse transcription reaction of a smRNA as defined above. One particular cDNA consists of SEQ ID No 3 or SEQ ID No 4.

In another embodiment, the invention concerns a vector comprising a sequence encoding the smRNA of the invention. The term "vector" refers to a DNA molecule originating from a virus, a bacteria, or the cell of a higher organism into which another DNA fragment of appropriate size can be integrated without loss of the vectors capacity for self-replication; a vector introduces foreign DNA into host cells, where it can be reproduced in large quantities. Examples are plasmids, cosmids, and yeast artificial chromosomes; vectors are often recombinant molecules containing DNA sequences from several sources.

Preferred vectors are plasmids, more specifically plasmids which comprise the construct as shown in figure 1, notably the sequence SED ID No 7 or SEQ ID No 9 and 11. The invention also relates to a vector as depicted in figure 2. More particularly, the invention is directed to a vector as mentioned above which further comprise any sequence corresponding to SEQ ID No 9, 10 and 11 preceded by a sequence corresponding to a RNA polymerase promoter, for example RNA polymerase T7 promoter (sequence ID No 8) and referred as to "DNA probe DNAΣ" (see figure 3).

In still another embodiment, the invention is aimed at a kit for quantification of mRNAs of a biological sample comprising a smRNA, a vector or a DNA probe as defined above. Such kit may further comprise primers of SEQ ID No 5 and 6.

The invention also relates to the use of a smRNA, a vector or a DNA probe as defined above for calibrating target mRNAs during quantification in RT-PCR reaction, more particularly in frame with Q-RT-PCR or DD-RT-PCR, which may optionally be practiced with DNA microarrays.

The invention also relates to the use of a smRNA, a vector or a DNA probe as defined above for calibrating target mRNAs in Northern blot analysis.

The invention is further illustrated in the examples below.

### EXAMPLE 1: Calibration of the reverse transcription using a synthetic messenger RNA (smRNA) obtained from a synthetic DNA probe (DNA∑)

### 1.1 Characteristics of the DNA probe: DNAΣ

- Name of the DNA probe : DNAΣ
- Total Length : 182 bp
- Insertion of a restriction site for BSM I allowing the linearization of the vector just after the poly A sequence
- Presence of the T7 promoter allowing the in vitro transcription of a synthetic messenger RNA (smRNA)

### 1.2 Construction of the DNA probe

- Insertion of a 101 bp fragment (shown below) in the pGEM®-T Easy Vector with a T4 DNA ligase (Promega). 
5'TTCTTCGACTCACTGCAGACTACTGATGGAATGACGTAGTACGAATAC
TCGACTGGTCTCAACATGAAAAAAAAAAAAAACGCATTCAACCTGTCTG
ACTA 3' (SEQ ID No 11).
- Linearization of the cloned plasmid with the Aat II and Sph I restriction enzymes (New England BioLabs®Inc).

3- Insertion of a 27 bp fragment (shown below) with a T4 DNA ligase (Promega).

5'-CGGGACAAGAAGGTGGAAGACGTCATG-3' (SEQ ID No 9).

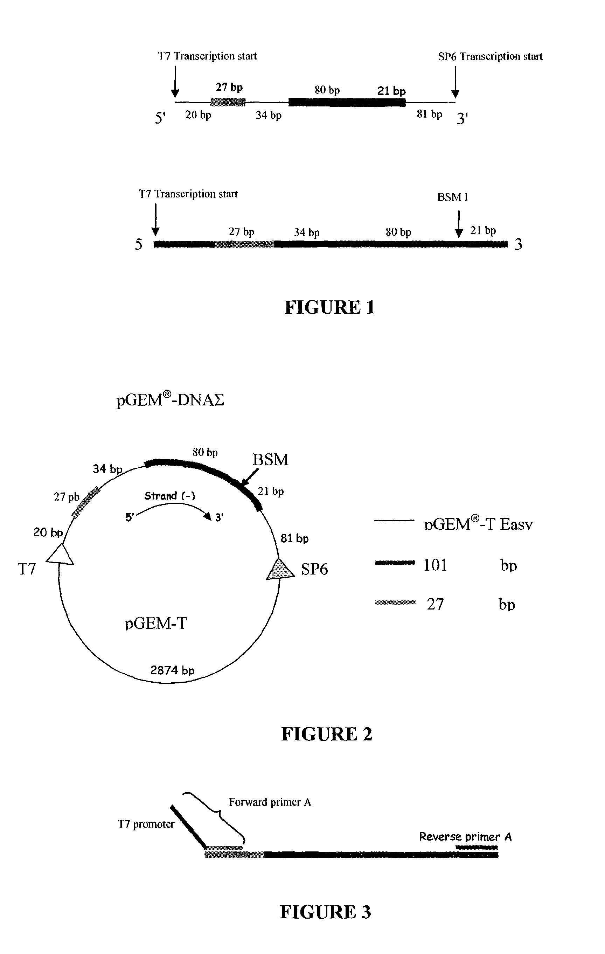

The resulting plasmid referred as pGEM®-DNA∑ is shown in Figure 2.

### 1.3 Synthesis of the DNA probe "DNAΣ"

PCR amplification of the plasmid pGEM®-DNA∑ using:
- a mix of HotStarTaq DNA polymerase (Qiagen) and ProofStar DNA polymerase (Qiagen).
- Forward primer A containing the T7 promoter: 
5'TAATACGACTCACTATAGGGCGGACAAGAAGGTGGAAG3' (SEQ ID
No 12)
- Reverse primer A:

5’TAGTCAGACAGGTTGAATGCG3’ (SEQ ID No 13)

Sequence of the DNA probe "DNAΣ"

5'TAATACGACTCACTATAGGGCGGACAAGAAGGTGGAAGACGTCATG
CTCCCGGCCGCCATGGCGGCCGCGGAATTCGATTTCTTCGACTCACTG
CAGACTACTGATGGAATGACGTAGTACGAATACTCGACTGGTCTCAACA
TGAAAAAAAAAAAAAACGCATTCAACCTGTCTGACTA3' (SEQ ID No 7).

TAATACGACTCACTATAGGG = sequence of the T7 promoter (SEQ ID No 8)
CGGGACAAGAAGGTGGAAGACGTCATG = 27 bp insert (SEQ ID No 9)
CTCCCGGCCGCCATGGCGGCCGCGGGAATTCGAT = 34 bp from pGEM®-T
Easy sequence (SEQ ID No 10)

TTCTTCGACTCACTGCAGACTACTGATGGAATGACGTAGTACGAATACT
CGACTGGTCTCAACATGAAAAAAAAAAAAAACGCATTCAACCTGTCTGA
CTA = 101 bp insert (SEQ ID No 11).

### Example 2: In vitro transcription of the synthetic mRNA using the plasmid pGEM®-DNA∑

- Linearization of the plasmid pGEM® -DNAΣ with the BSM I restriction enzyme (New England BioLabs®*Inc*); see Figure 1.
- *In vitro* transcription with T7 RNA polymerase using T7-MEGAshortscriptTM (Ambion ®).

5’GGGCGAAUUGGGCCCGACGUCGGACAAGAAGGUGGAAGACGUCAU
GCUCCCGGCCGCCAUGGCGGCCGCGGAAUUCGAUUUCUUCGACUCAC
UGCAGACUACUGAUGGAAUGACGUAGUACGAAUACUCGACUGGUCUC
AACAUGAAAAAAAAAAAAAA 3’ (SEQ ID No 1)

Synthetic poly A mRNA #1 (161 nt)

### Example 3: In vitro transcription of the synthetic mRNA using the DNA probe « DNAΣ»

- Digestion of the DNA probe « DNAΣ» with the BSM I restriction enzyme (New England BioLabs®*In*c); see Figure 1.
- *In vitro* transcription with T7 RNA polymerase using T7-MEGAshortscript™ (Ambion ®).

5'UAAUACGACUCACUAUAGGGCGGACAAGAAGGUGGAAGACGUCAU
GCUCCCGGCCGCCAUGGCGGCCGCGGAAUUCGAUUUCUUCGACUCAC
UGCAGACUACUGAUGGAAUGACGUAGUACGAAUACUCGACUGGUCUC
AACAUGAAAAAAAAAAAAAA 3' (SEQ ID No 2).

Synthetic poly A mRNA #2 (161 nt)

### Example 4: RT-PCR of both synthetic mRNAs #1 and #2

4.1 Reverse transcription of synthetic poly A mRNAs #1 (SEQ ID No 1) and #2 (SEQ ID No 2) with oligo dT using M-MLV Reverse Transcriptase RNase H Minus, point mutant (Promega) leading to : 
Synthetic cDNA #1
5'GGGCGAATTGGGCCCGACGTCGGACAAGAAGGTGGAAGACGTCATG
CTCCCGGCCGCCATGGCGGCCGCGGGAATTCGATTTCTTCGACTCACTG
CAGACTACTGATGGAATGACGTAGTACGAATACTCGACTGGTCTCAACA
TGAAAAAAAAAAAAAA 3' (SEQ ID No 3).
 and 
Synthetic cDNA #2
5'TAATACGACTCACTATAGGGCGGACAAGAAGGTGGAAGACGTCATG
CTCCCGGCCGCCATGGCGGCCGCGGGAATTCGATTTCTTCGACTCACTG
CAGACTACTGATGGAATGACGTAGTACGAATACTCGACTGGTCTCAACA
TGAAAAAAAAAAAAAA 3' (SEQ ID No 4).
4.2 Amplification of synthetic cDNAs #1 and #2 using PCR on the LightCycler® (Roche) using the QuantiTectTM SYBR® Green PCR kit (Qiagen) or the LC FastStart DNA Master SYBR Green I (Roche).

Primer III forward (19 nt): 5' CGG GAC AAG AAG GTG GAA G 3' (SEQ ID No
5).

Primer III reverse (22 nt): 5' AGT CTG CAG TGA GTC GAA GAA A 3' (SEQ ID
No 6)

The amplified fragment from both synthetic cDNA #1 and cDNA #2 (82 pb) with primer pair III produces the following sequence:

5’ GGGACAAGAAGGTGGAAGACGTCATGCTCCCGGCCGCCATGGCGGCC
GCGGGAATTCGATTTCTTCGACTCACTGCAGACT 3’ (SEQ ID No 14).

### Example 5: Calibration of the RT reaction using smRNAs of the invention in real-time PCR.

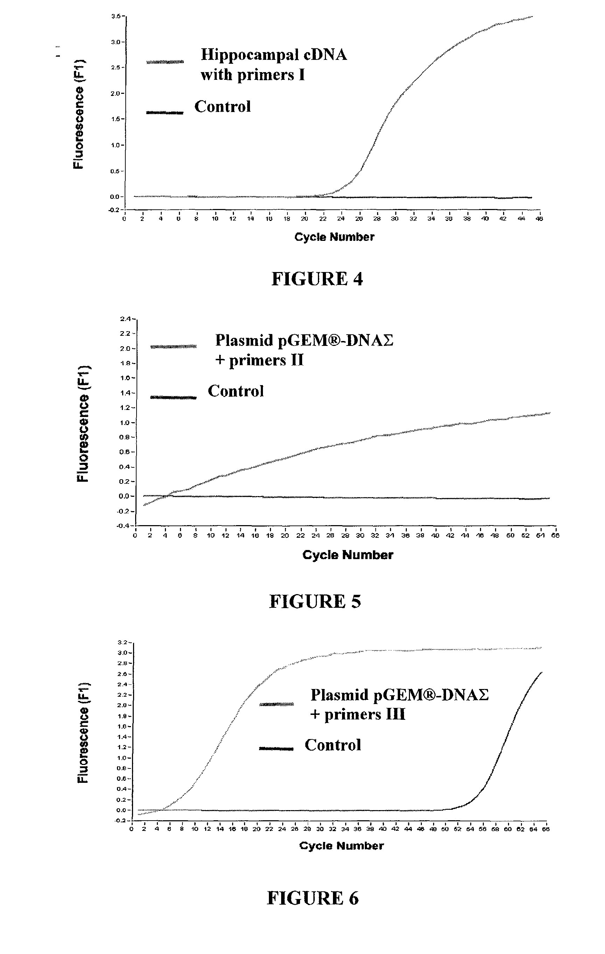

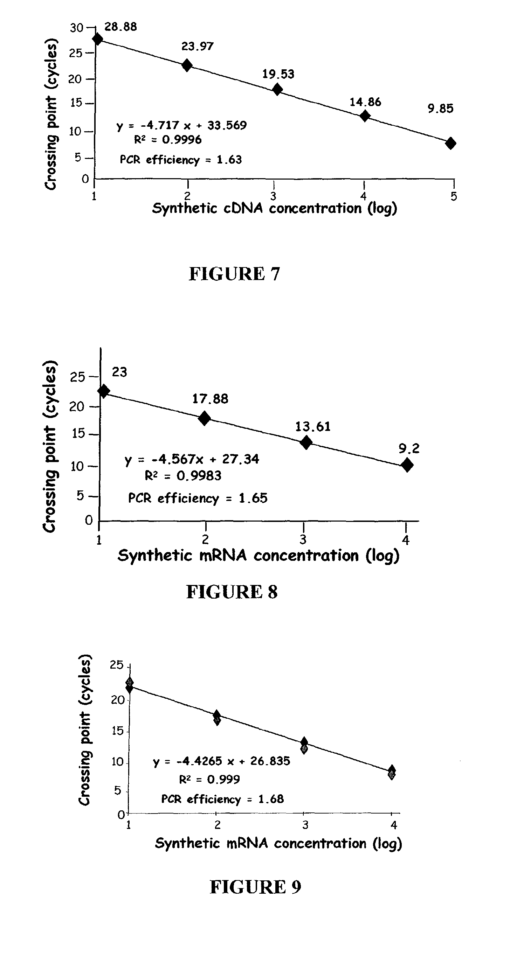

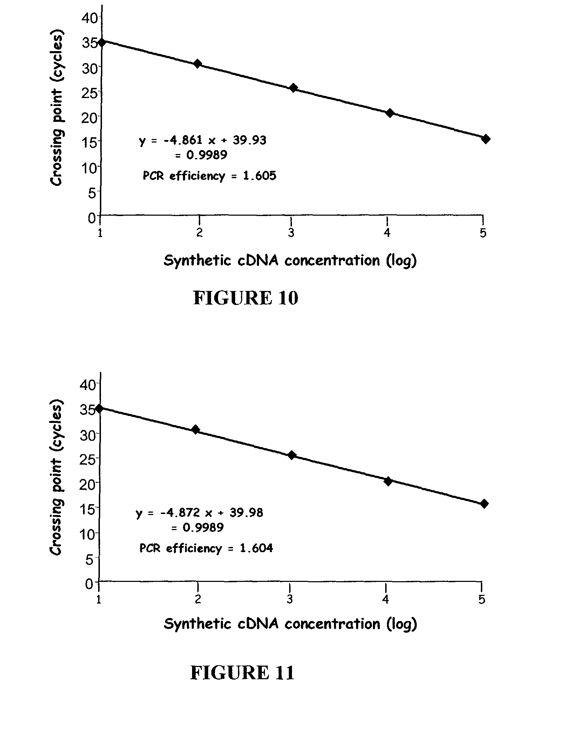

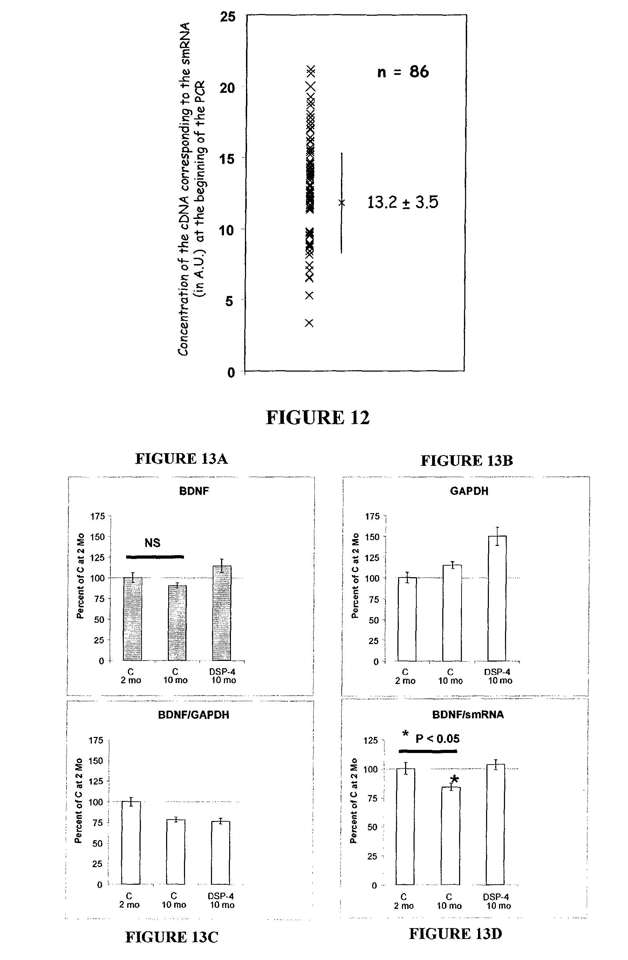

5.1 We tested several primers to optimize the sequence of the smRNA as depicted above. The goal was to obtain segments within the sequence which are complementary to pairs of primers which will not dimerize during the reaction while being specific to the smRNA.
   Among the sequences tested, we show here below three examples illustrating the importance of the presence of such segments within the smRNA of the invention. 
Pair I: primer forward (20 nt) 5' AATTGGGCCCGACGTCGCAT 3' SEQ ID No 15
and primer reverse (20 nt) 5' CATGTTGAGACCAGTCGAGT 3' SEQ ID No 16
 
Pair II: primer forward (19 nt) 5' CGGGACAAGAAGGTGGAAG 3' SEQ ID No
17 and primer reverse (20 nt) 5' TCATGTTGAGACCAGTCGAG 3' SEQ ID No 18
 
Pair III: primer forward (19 nt) 5' CGGGACAAGAAGGTGGAAG 3' SEQ ID No
5 and primer reverse (22 nt) 5' AGTCTGCAGTGAGTCGAAGAAA 3' SEQ ID No
6
   The left and right primers I, II and III were chosen to minimize as much as possible the PCR priming primer-dimers using the "primer 3" software.
   Regarding primers I, control experiments without cDNA show the absence of any amplification, confirming the absence of primer dimerization (curve control, Figure 4). However, these primers did not provide satisfying specificity since (in absence of SmRNA during the RT), they recognized cDNAs obtained from hippocampal tissue (curve cDNA, Figure 4).
   Based on these results, it was necessary to design different specific primers. Several specific software, including Primer3, did not allow us to design other primers matching the various cautious required such as the absence of self complementarity and melting temperatures. Therefore, we have inserted a 27 bp fragment in the pGEM®-T Easy Vector with a T4 DNA ligase (Promega) in order to design other primers.
   The left and right primers II were chosen to minimize as much as possible the PCR priming primer-dimers. Control experiments without cDNA show the absence of any amplification, which confirms the absence of primer dimerization (curve control, Figure 5). However, the profile of the amplification curve of the pGEM®-DNA∑, that the primer pair has a weak affinity for the sequence. Other specific primers have thus been designed.
   In case of the primers III, control experiments. without cDNA containing the sequence to amplify, show the absence of any amplification, which confirms the absence of primer dimerization (curve control, Figure 6). Furthermore, the amplification curve shows that primers III have a high affinity for the sequence of the pGEM®-DNA∑, which contains the sequence to amplify.
   Using such tests, one can routinely obtain variants of the smRNA of SEQ ID No 1 and corresponding primers optimized for use as mentioned in the present invention.
**5.2 Calibration using primers III and the smRNA of SEQ ID No 1.**
   5.2.1 Calibration of the amplification of the synthetic cDNA using the LightCycler® (Roche) and the QuantiTectTM SYBR® Green PCR kit (Qiagen). Several dilutions of the purified synthetic cDNA were amplified with specific primer pair III and the crossing point was determined.
      We evidence that the starting concentration of synthetic cDNA before amplification is linearly correlated with the value of the crossing point (validation #1, Figure 7).
   5.2.2 Increasing amounts of synthetic mRNA #1 were reverse transcribed and amplified with specific primer pair III using QuantiTectTM SYBR® Green PCR kit (Qiagen) on the LightCycler®
      We evidence that the amplification of the synthetic cDNA, following reverse transcription of the synthetic mRNA, is dependant of the concentration of the synthetic mRNA #1 reverse transcribed (validation #2, Figure 8).
   5.2.3 Increasing amounts of synthetic mRNA #1 were reverse transcribed together with 500 ng of total hippocampus RNA of either control or pilocarpine-treated rats. Synthetic cDNA were amplified as above.
      We evidence that the dilution of the synthetic mRNA #1 either within total tissue RNA from control or pilocarpine-treated rats does not affect the PCR efficiency (1.68) for synthetic mRNA #1 (validation #3, Figure 9).
   5.2.4 Calibration of the amplification of the synthetic cDNA using the LightCycler® (Roche) and LC FastStart DNA Master SYBR Green I (Roche). Several dilutions of the purified synthetic cDNA were amplified and the crossing point was determined.
      This calibration curve using the LC FastStart DNA Master SYBR Green I (Roche) is very similar to that obtained with the QuantiTect SYBR Green PCR kit (Qiagen) (see validation # 1, Figure 7), demonstrating that the synthetic cDNA can be equally amplified with the LightCycler using both kits (validation #4, Figure 10).
   5.2.5 Calibration of the amplification of the synthetic cDNA using the LightCycler® (Roche) and LC FastStart DNA Master SYBR Green I (Roche). Several dilutions of the purified synthetic cDNA were amplified and the crossing point was determined. In this experiment, the serial dilutions have been performed with a constant concentration of cDNAs obtained from reverse transcription of total brain mRNAs, instead of water (validations # 1 and 4, Figures 7 and 10 respectively).
      We evidence that the amplification of the synthetic cDNA is independent of the diluent used (water in validation # 4 = Figure 10 versus brain cDNAs in validation # 5 = Figure 11).
   5.2.6 Illustration of the variability of the RT efficiency performed in 86 samples of total RNA from mouse olfactory bulb (OB).
      500 ng of total RNA from 86 OB have been reverse transcribed together with 80 pg of smRNA #1. Reverse transcription has been performed in a PCR Express Thermocycler (Hybaid). cDNA corresponding to the smRNA #1 has then been quantitatively amplified with primer pair "III" using the LightCycler to control whether identical concentration of cDNA from smRNA was present in each sample after the reverse transcription step.
      Conclusion: The figure 12 shows a wide range of cDNA concentration after reverse transcription, indicating that the reverse transcription was not reproducible across the 86 samples.
   5.2.7 Demonstration that smRNA #1 is appropriate, instead of house-keeping gene GAPDH, to quantify specific mRNA following quantitative relative RT-PCR.
      500 ng of total RNA from rats (control 2 month old; control 10 month old; DSP-4 treated 10 month old) have been reverse transcribed together with 80 pg of smRNA #1. cDNAs corresponding to BDNF exon V, GAPDH and smRNA have been amplified using gene specific primers with the LightCycler. In each sample, BDNF was either corrected using either GAPDH value or smRNA value.
      Note that GAPDH cannot be used to normalize samples, since its expression is not constant among the three groups tested (Figure 13B). However, smRNA appears to highlight differences that could not been evidenced before normalization of the reverse transcription (Figure 13A vs. Figure 13D). Furthermore, note that normalization with GAPDH induces biased conclusions (Figure 13B vs. Figure 13D).
   5.2.8 Influence of the smRNA #1 addition to the RT reaction the relative variation in the expression of specific genes.
      Reverse transcription of 500 ng of total hippocampus RNA from either control or pilocarpine-treated rats. Piolcarpine treatment has been used to stimulate BDNF gene expression in the hippocampus. Exon 5 of the BDNF cDNA was amplified with specific primers and LC FastStart DNA Master SYBR Green I (Roche). BDNF mRNA concentration was quantified using PCR on the LightCycler and the standard curve equation: y = -3.7621 x + 31.648.

### BDNF mRNA concentration in:

- Control:: 31.5±3.7A.U.
- Pilocarpine:: 219.9 ± 36.9 A.U.
- **Pilocarpine =**: **6.98 X control**

Reverse transcription of 500 ng of total hippocampus RNA from either control or pilocarpine-treated rats, together with 80 pg of synthetic mRNA #1 added to the RT reaction mix. Exon 5 of the BDNF cDNA was amplified with specific primers and LC FastStart DNA Master SYBR Green I (Roche). BDNF mRNA concentration was quantified using PCR on the LightCycler and the standard curve equation: y = - 3.7621 x + 31.648.

Synthetic cDNA was amplified with specific primers and QuantiTect SYBR Green I PCR kit (Qiagen). Synthetic mRNA #1 concentration was quantified using PCR on the LightCycler and the standard curve equation: y = -4.717 x + 33.569.

### BDNF mRNA concentration in:

- Control:: 31.4 ± 1.1A.U.
- Pilocarpine:: 245.5 ± 26.2 A.U.

### Synthetic mRNA #1 concentration in:

- Control:: 1.627 ± 0.062 A.U.
- Pilocarpine:: 1.821 ± 0.053 A.U.

### BDNF mRNA / synthetic mRNA in:

- Control:: 19.3
- Pilocarpine:: 134.8
- **Pilocarpine =**: **6.98 X control**

### Conclusion:

The addition of the synthetic mRNA to the reverse transcriptase (RT) reaction mix does not interfere with the reverse transcription of endogenous mRNA.
The normalization of BDNF cDNA by the synthetic cDNA allows to recover the same induction profile in pilocarpine-treated rats than that observed without the addition of the synthetic mRNA to the RT reaction mix.

Therefore, we conclude that the addition of the smRNA of the invention into the RT reaction mix allows a reliable normalization of the RT reaction between samples.

## Claims

**1.** A method for quantifying products of reverse transcription reaction of mRNA extracted from a biological sample, wherein said method comprises the steps consisting of adding a synthetic messenger RNA (smRNA) which do not interfere with the reverse transcription of endogenous mRNA of said sample to a reaction mixture comprising mRNA extracted from said sample, which smRNA display less than 95%, 97% or 99% or preferably less than 99,50 % identity with any biological sequence, to perform a reverse transcription reaction and to calibrate the quantity of each target mRNA obtained after amplification with the quantity of the cDNA amplified corresponding to said smRNA.

**2.** A method according one of claims 1 and 2, wherein said smRNA is added in different reaction mixtures comprising one sample in different dilutions.

**3.** A method according to claim 1, wherein said smRNA further comprises a polyA segment.

**4.** A method according to one of claims 1 to 3, wherein said smRNA is about 80 nucleotide long or longer, preferably about 100 nucleotide long.

**5.** A method according to one of claims 1 and 2, wherein said smRNA is specifically amplified with a pair of primers designed to avoid primer dimerization.

**5.** A method according to one of claims 1 to 4 consisting of quantitative RT-PCR, wherein quantification of target mRNAs is performed by means of normalization with said smRNA.

**6.** A method according to one of claims 1 to 4 consisting of DD-RT-PCR, wherein quantification of target mRNAs is performed by means of normalization with said smRNA.

**7.** A method according to one of claims 5 and 6 which is performed on DNA microarrays.

**8.** A method according to claim 7, wherein cDNA corresponding to the smRNA is amplified and spotted onto said microarrays.

**9.** A method according to one of claims 1 to 4 consisting of Northern blotting wherein said smRNA is pooled with samples.

**10.** A method according to one of claims 1 to 9, wherein said smRNA consists of a SEQ ID No 1 or SEQ ID No 2 or sequences deriving thereof.

**11.** A method according to claim 10, wherein said smRNA is reversed transcribed into cDNA of SEQ ID No 3 using a pair of primer consisting of SEQ ID No 4 and SEQ ID No 5.

**12.** A synthetic messenger RNA (smRNA) designed for normalization of reverse transcription reaction of mRNAs of a biological sample, wherein said smRNA do not interfere with the reverse transcription of endogenous mRNA of said sample, which smRNA display less than 95% or 99% identity with any biological sequence, comprise a poly A segment and is about 80 nucleotide long or longer, preferably about 100 nucleotide long.

**13.** A smRNA according to claim 12 which further comprise at least two segments which are complementary to at least two primers that are designed to avoid primer dimerization.

**14.** A smRNA according to one of claims 12 and 13 consisting of SEQ ID No 1 or SEQ ID No 2.

**15.** A smRNA according to claim 13 comprising two segments which are complementary to at least two primers of SEQ ID No 5 and SEQ ID No 6.

**16.** A primer or probe selected from of SEQ ID No 5 and SEQ ID No 6.

**17.** A cDNA obtained from a reverse transcription reaction of a smRNA according to one of claims 12 to 15.

**18.** A cDNA according to claim 17 which consist of SEQ ID No 3 or SEQ ID No 4.

**19.** A vector, more particularly a plasmid, comprising a sequence encoding the smRNA of one of claims 12 to 15.

**20.** A vector according to claim 19 which further comprise any sequence corresponding to SEQ ID No 9, 10 and 11 preceded by a sequence corresponding to a RNA polymerase promoter, in particular RNA polymerase T7 promoter (sequence ID No 8) and referred as to "DNA probe DNAΣ" (see figure 3).

**21.** A vector according to claim 19 or 20 which comprises the construct as shown in figure 1, notably the sequence SED ID No 7 or SEQ ID No 11.

**22.** A vector according to claim 21 as depicted in figure 2.

**23.** A kit for quantification of mRNAs of a biological sample comprising a smRNA according to one of claims 12 to 15, or a vector according to one of claims 19 to 22.

**24.** A kit according to claim 23 further comprising primers of SEQ ID No 5 and 6.

**25.** The use of a smRNA according to one of claims 12 to 15 or a vector according to one of claims 19 to 22 for calibrating target mRNAs during quantification in RT-PCR reaction.

**26.** The use according to claim 25 in Q-RT-PCR or DD-RT-PCR.

**27.** The use according to claims 25 or 26 in frame with DNA microarrays.

**28.** The use of a smRNA according to one of claims 12 to 15 or a vector according to one of claims 19 to 22 for calibrating target mRNAs in Northern blot analysis.
